# EUROPEAN PATENT APPLICATION

(11) **EP 3 779 443 A1**
(43) Date of publication of application: **17.02.2021**
(21) Application number: 19781906.3
(22) Date of filing: 05.04.2019
(51) Int. Cl.: G01N 33/564, G01N 33/558, G01N 33/68

(54) **NOVEL EPITOPE OF IMMUNOGLOBULIN E, ANTIBODY BINDING THERETO, AND KIT FOR ANALYZING IMMUNOGLOBULIN E IN SAMPLE CONTAINING SAME**

(30) Priority: 06.04.2018 KR 20180040605
(71) Applicant: SLSBIO CO., LTD., Suwon-si, Gyeonggi-do 16229 (KR)
(72) Inventor: KIM, Bong Hui, Hwaseong-si, Gyeonggi-do 18442 (KR); PARK, Eun Young, Suwon-si, Gyeonggi-do 16649 (KR); JANG, Ha Kyung, Yongin-si, Gyeonggi-do 16993 (KR); HONG, Kwang Won, Suwon-si, Gyeonggi-do 16509 (KR)
(74) Representative: Macquet, Christophe
(86) International application number: PCT/KR2019/004104
(87) International publication number: WO 2019/194656

(57) **Abstract**

Provided are a peptide consisting of an amino acid sequence of SEQ ID NO: 3, which is a novel epitope of immunoglobulin E (IgE), and an anti-IgE antibody binding to the peptide, in which the peptide and the antibody may be applied for diagnosing an allergic disease or research on a therapeutic agent therefor. In addition, provided are a kit for analyzing IgE in a sample containing the antibody and a method of diagnosing an allergic disease using the kit, which may perform qualitative analysis to determine the presence or absence of a typical inhalant and food allergies with high incidence in Koreans, and diagnose an allergy within a short period after blood collection.

## Description

### [Technical Field]

The present invention relates to a novel epitope of immunoglobulin E, an antibody binding thereto and a kit for analyzing immunoglobulin E in a sample, which includes the antibody.

### [Background Art]

An allergy is a sudden and hypersensitive change in a living body, caused by an antigen-antibody reaction occurring when a living organism is in contact with a foreign substance, and the foreign substance causing this is an allergen. Among various types of allergic responses, an allergy involving immunoglobulin E (IgE) is called type I allergy.

The type I allergy is caused by the release of a compound occurring during a hypersensitivity response due to the stimulation of mast cells or basophils when allergens are attached to immunoglobulins attached to the surfaces of the mast cells or basophils. Generally, allergic symptoms include asthma, eczema, hives, dermatitis, and rhinitis.

Since total IgE antibodies and allergen-specific IgE antibodies may increase in the blood of an individual with allergies, the measurement of total IgE concentration or allergen-specific IgE concentration in serum is used in a useful test for distinguishing symptoms caused by a typical allergic response from diseases similar to an allergy. Generally, the concentration of total IgE antibodies in cord blood at birth is approximately 1 IU/ml (2.4 ng = 1 IU), and gradually increases with age and thus is maintained at generally 83.3 IU/ml (200 ng/ml) or less in adults.

The identification of a specific allergen provides information for tracking a disease, and the allergen includes mites, pollen, animal epithelial cells and various foods. The measurement of IgE antibodies in serum will provide useful clinical information for the treatment of allergic patients.

### [Disclosure]

### [Technical Problem]

One aspect of the present invention provides a peptide consisting of an amino acid sequence of SEQ ID NO: 3, which is an epitope of immunoglobulin E (IgE).

Another aspect of the present invention provides an anti-IgE antibody binding to the peptide.

Still another aspect of the present invention provides a kit for analyzing IgE in a sample, which includes the antibody.

Yet another aspect of the present invention provides a device for diagnosing or predicting a prognosis of allergies, which includes the kit; and a specimen loading part.

Yet another aspect of the present invention provides a method of diagnosing an allergic disease, which includes: collecting the blood of a subject and mixing it with a specimen diluent; and dropping the dilution into the specimen loading part to perform a reaction.

### [Technical Solution]

One aspect of the present invention provides a peptide consisting of an amino acid sequence of SEQ ID NO: 3, which is an epitope of IgE.

The epitope may be conjugated with an immunogenicity-increasing substance or carrier, and the immunogenicity-increasing substance or carrier may be, for example, Keyhole Limpet Hemocyanin (KLH).

The peptide may consist of an amino acid sequence of a part of the amino acid sequence of IgE, and may be used as an epitope of IgE, that is, a binding site of the antibody. The peptide has excellent binding strength when binding to the anti-IgE.

The amino acid sequence of the epitope of IgE may be a peptide consisting of a 12- to 15-amino-acid sequence, and more particularly, a peptide consisting of the amino acid sequence of SEQ ID NO: 3.

The term "peptide" refers to a polymer consisting of two or more amino acids linked by amide bonding or peptide bonding. The polypeptide may consist of the amino acid sequence of SEQ ID NO: 3. The polypeptide may include peptides having approximately 70% or more, approximately 75% or more, approximately 80% or more, approximately 85% or more, approximately 90% or more, approximately 92% or more, approximately 95% or more, approximately 97% or more, approximately 98% or more, or approximately 99% or more sequence homology with the amino acid sequence of SEQ ID NO: 3.

In addition, to acquire better chemical stability, strengthened pharmacological properties (half-life, absorption, titer, efficacy, etc.), changed specificity (e.g., a broad spectrum of biological activity), and reduced antigenicity, a protecting group may be bound to the N- or C-terminus of the peptide. The protecting group may be an acetyl group, a fluorenyl methoxy carbonyl group, a formyl group, a palmitoyl group, a myristyl group, a stearyl group or polyethylene glycol (PEG), but may include any component that can improve modification of a peptide, and particularly, the stability of a peptide without limitation.

The term "stability" may refer to *in vivo* stability that protects the peptide from an attack of a protease *in vivo* as well as storage stability (e.g., room temperature storage stability).

Moreover, the peptide may further include an amino acid sequence prepared for a specific purpose for a targeting sequence, a tag or a labeled residue.

The term "homology" represents the degree of similarity with a wild-type amino acid sequence, and such homology comparison may be performed using a comparison program widely known in the art, and the homology between two or more sequences may be calculated as percentage (%).

The peptide may be derived from nature, and may be obtained by various peptide synthesis methods widely known in the art. In one example, the peptide may be prepared using polynucleotide recombination and a protein expression system, or using an in vitro synthesis method through chemical synthesis such as peptide synthesis and a cell-free protein synthesis method. In addition, as an example, the peptide may be a polypeptide, a plant-derived tissue or cell extract, or a product obtained from culture of microorganisms (e.g., bacteria or fungi, and particularly, yeast), and specifically, may be derived from IgE, and more specifically, a part of the amino acid sequence of IgE.

In addition, one aspect of the present invention provides a gene encoding the epitope, and the gene encodes a peptide consisting of the amino acid sequence of SEQ ID NO: 3.

In addition, one aspect of the present invention provides a vector including the gene encoding the epitope.

The vector may be introduced into a host to be used by the host to express the gene, and thus is used in preparation of the peptide. In addition, the vector may be one in which the gene introduced into an expression vector known in the art.

The vector may be a vector in which expression regulatory sequence and or a nucleotide sequence encoding a promoter and a signal sequence, and the vector may include an antibiotic resistant marker to select a vector-introduced host, and the marker may be inherent to the vector or derived from outside.

Another aspect of the present invention provides an anti-IgE antibody binding to the peptide.

The antibody may recognize the peptide as an epitope to bind to a corresponding part thereof, so that it can specifically bind to IgE. The antibody has not only excellent binding strength to the peptide, but also excellent binding strength to IgE. The antibody may be prepared by a known antibody preparation method, and may be prepared, specifically, from cells producing a polyclonal antibody or monoclonal antibody.

In still another aspect of the present invention provides a kit for diagnosing or analyzing a prognosis of an allergic disease, which enables qualitative analysis of allergen-specific IgE within 10 to 40 minutes after a sample is brought into contact with a subject, the kit including the antibody.

The kit may include an allergen and a membrane coated with the antibody. Specifically, the allergen may be a typical inhalant or food allergy with high incidence in Koreans. For example, the allergen may be a house dust mite, *Lolium perenne* L., oak, mugwort, *Humulus japonicus,* garlic, pork, tuna, fungus, a dog, sesame, peanut, beef, *Anthoxanthum odoratum* L., shrimp, apple, a cat, alder, birch, soybean, walnut, cod, *Dactylis glomerata,* milk, peach, potato, *Ambrosia artemisiifolia* L., egg white, wheat, crab, barley, reed, a cockroach, buckwheat or house dust. The antibody is as described above.

The membrane may be a material which can include a marker, and may be, for example, nitrocellulose, nylon, polyvinylidene fluoride (PVDF), glass or plastic.

In addition, the kit according to one embodiment may be for immunochromatography. Specifically, based on the principle of immunochromatography, the kit may be to detect the presence or absence of a specific IgE antibody against the allergen through human serum or plasma migration on a nitrocellulose membrane where each allergen is immobilized on a test line.

The term "immunochromatographic assay (ICA)" is a test method applying immunochemistry and thin-layer chromatography technology, and an application of specific reactivity of an antibody for an antigen, the color development characteristic and mobility of colloidal gold, and movement of a specimen and a molecule by a capillary force on a porous nitrocellulose membrane.

That is, in the kit according to one embodiment, a specimen containing a specific IgE antibody against an allergen reacts with an anti-IgE gold conjugate, and then migrates on a porous nitrocellulose membrane where the allergen is immobilized on a test line region and mouse IgG is immobilized on a control line region. When a specific IgE antibody for each allergen which is immobilized on the membrane is present in the specimen among antigen (specimen)-anti-IgE antibody gold conjugates migrating along the membrane, the gold antigen-antibody conjugates pass through a region to which each allergen is immobilized, and thus a red-violet band is formed at a corresponding location, and in addition, a goat anti-mouse IgG gold conjugate is captured by an antigen-antibody reaction with mouse IgG at the control line site, the control line may be designed to enable always color development.

Still another aspect of the present invention provides a device for diagnosing or predicting a prognosis of an allergy, which includes the kit; and a specimen loading part. The specific details of the kit are as described above.

The specimen loading part may contain a developing solution, and the developing solution may be, for example, bovine serum albumin, goat serum, sodium chloride, potassium chloride, Polysorbate 20, sodium azide, tertiary distilled water, ProClin 300, triaminomethane or a mixed solution thereof.

Yet another aspect of the present invention provides a method of diagnosing an allergic disease, which includes collecting the blood of a subject and mixing it with a specimen diluent; and dropping the dilution into a specimen loading part to perform a reaction for 10 to 40 minutes.

The method according to one embodiment includes collecting the blood of a subject and mixing it with a specimen diluent. The blood may be collected by a known method, and the volume of the blood mixed with the diluent may be 0.5 to 2 mL. For example, the volume may be 0.5 to 1.8 mL, 0.5 to 1.5 mL, 0.7 to 2 mL, 0.7 to 1.5 mL, 0.8 to 1.4 mL, or 1 to 1.5 mL. Here, when the volume of the blood is less than the above range, the antigen-antibody reaction may not sufficiently occur, and thus diagnostic accuracy may decrease. The specimen diluent may be prepared by diluting the specimen 2- to 64-fold, for example, 2-, 4-, 8-, 16-, 32- or 64-fold.

In the method according to one embodiment, the blood may be plasma or serum. When plasma or serum is mixed with the specimen diluent, the volume of the plasma or serum may be 0.1 to 1 mL. For example, the volume may be 0.1 to 1 mL, 0.1 to 0.9 mL, 0.1 to 0.8 mL, 0.2 to 1 mL, or 0.2 to 0.8 mL. Here, when the volume of the plasma or serum is less than the range, due to an insufficient antigen-antibody reaction, diagnostic accuracy may decrease.

The method according to one embodiment includes dropping the dilution into the specimen loading part to perform a reaction for 10 to 40 minutes. Specifically, 1 to 5 drops, for example, 1 to 5, 1 to 4, 2 to 5, or 2 to 4 drops of the dilution may be dropped into the specimen loading part using a dropper. Here, the reaction may proceed for 10 to 40 minutes, for example, 10 to 40, 10 to 35, 10 to 30, 15 to 40, 15 to 35, or 15 to 30 minutes. Here, when the reaction time is less than the above range, the specimen diluent may not sufficiently react with IgE of the specimen loading part.

The method according to one embodiment may further include confirming the presence or absence of an allergen-specific antibody using a card for confirming a result in a device in which the reaction is completed.

### [Advantageous Effects]

A peptide and an antibody according to one aspect are epitopes of IgE, and can be applied in immunology, or diagnosis of an allergy or research on a therapeutic agent therefor. In addition, a kit including the antibody can be used to qualitatively analyze the presence or absence of typical inhalant and food allergies with high incidence in Koreans, and visually confirm a result within a short time after blood collection. In addition, it has an advantage that it can be applied to children who are difficult to collect blood because it is possible to minimize a specimen amount required for a test.

### [Description of Drawings]

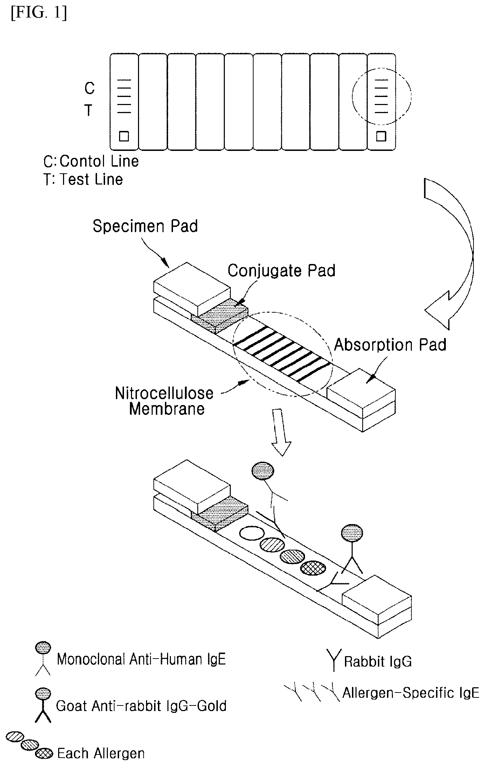

FIG. 1 is a schematic diagram of a reaction of a kit according to one aspect.

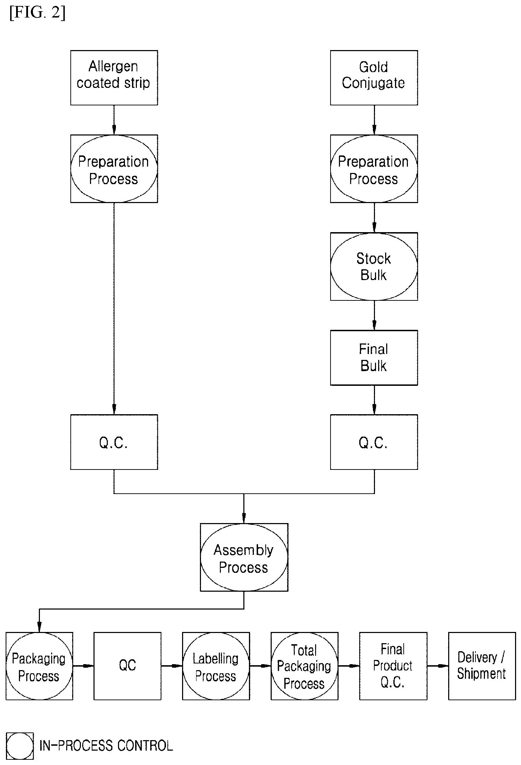

FIG. 2 is a schematic diagram illustrating a process of manufacturing a device according to one embodiment.

### [Modes of the Invention]

Hereinafter, to help in understanding the present invention, exemplary examples will be suggested. However, the following examples are merely provided to more easily understand the present invention, and not to limit the present invention.

### [Examples]

### Example 1. Confirmation of novel IgE epitope

### (1) Preparation of polyclonal antibody and confirmation of titer

100 µL of an antigen (IgE pep-40) and an equal amount of a complete adjuvant were mixed to prepare an antigen emulsion, and the resulting emulsion was administered subcutaneously to a 6-week-old female mouse (BALB/C) to induce an immune response. After primary administration, 100 µL of the same antigen and an equal amount of an incomplete adjuvant were administered subcutaneously to the mouse every two weeks (Table 1).

**[Table 1]**

| Degree | Antigen | Concentration (mg/mL) | Dose (µg) | |
|---|---|---|---|---|
| Primary | Antigen (IgE pep-40) | 1 | 75 | + **complete adjuvant** (v/v 1:1 mixture) |
| Secondary | Antigen (IgE pep-40) | 1 | 75 | + **incomplete adjuvant** (v/v 1:1 mixture) |
| Tertiary | Antigen (IgE pep-40) | 1 | 75 | + **complete adjuvant** (v/v 1:1 mixture) |

After performing antigen administration three times, a small amount of serum was collected from a tail vein of the mouse to confirm the titer of a polyclonal antibody in the serum through an ELISA test. Specifically, 2 µg/mL of the antigen (IgE pep-40) was reacted at 100 µL per well and 4 °C for 18 hours, followed by coating. The next day, a reaction (blocking) was performed using a blocking buffer at room temperature for 1 hour. Subsequently, the serum obtained from the mouse blood was diluted to the dilution-fold and then dispensed into wells at 100 µL, followed by a reaction at room temperature for 1 hour. After the reaction was completed, the plate was washed with PBST, goat anti-mouse IgG-HRP in which HRP was conjugated was diluted 1:2000 and dispensed into wells at 100 µL, followed by a reaction at room temperature for 1 hour. Afterward, the plate was washed with PBST, a TMB solution was dispensed to react for 15 minutes, and then the reaction was terminated using a reaction stop solution. Absorbance was measured at 450/620 nm to confirm the titer in serum (Table 2).

**[Table 2]**

| Serum concentration | Distinction | Control | NC1 | IgE pep-40 |
|---|---|---|---|---|
| x 100 | A | 0.203 | 0.087 | 2.576 |
| x 200 | B | 0.084 | 0.037 | 2.195 |
| x 400 | C | 0.03 | 0.014 | 2.001 |
| x 800 | D | 0.013 | 0.009 | 1.637 |
| x 1600 | E | 0.009 | 0.006 | 1.119 |
| x 3200 | F | 0.006 | 0.006 | 0.583 |
| x 6400 | G | 0.005 | 0.005 | 0.332 |
| x 12800 | H | 0.005 | 0.005 | 0.117 |

### (2) Preparation of hybridoma cells

It was confirmed that an antibody having a high titer was produced based on the titer in the serum of a mouse confirmed through an experiment for confirming an antibody titer, and lymphocytes of the mouse was isolated to perform cell fusion. The spleen of the mouse in which an immune response was induced was extracted without damage, washed with Dulbecco/Vogt modified Eagle's minimal essential medium (DMEM), lymphocytes were isolated and added to a 60 mm dish containing DMEM, followed by separation into single cells. Afterward, red blood cells mixed with the lymphocytes were removed using an RBC lysis buffer and washed with DMEM, and prepared myeloma cells (SP2/0 Ag 14 - ATCC #CRL-1581) were mixed with the lymphocytes at 1L5 based on a cell count. Subsequently, 1.7 mL of PEG1500 was added to the mixed cells to induce cell fusion, and the cells were seeded into a 96 well plate at 200 µL per well, and then incubated in a CO₂ incubator. Two days after cell fusion, 50% per well was replaced with a hypoxanthine-aminopterin thymidine (HAT) medium, and after 12 days, colonies were identified and then ELISA (Abs: 450 nm) was performed to determine whether the colonies were reacted with the IgE pep-40 protein peptide (antigen for ELISA). Among the results of ELISA, when an O.D value is 1.0 or more, the corresponding result was set to be positive, and monoclonal cells were selected.

### (3) Selection of hybridoma cell line producing monoclonal antibody

To select cells specifically responding to IgE pep-40 from the prepared fusion cell groups and confirm whether antibodies are produced, screening was performed using ELISA.

On day 12 after cell fusion, the medium was exchanged, and an ELISA test was performed using the exchanged medium as a primary antibody. After the ELISA test, a well showing positive for a corresponding antigen was selected, and transferred to a 24-well plate for incubation. The hybridoma cell line cultured in the 24-well plate was subjected to an ELISA test again by the same method to confirm an antibody titer. Absorbance (O.D. value) of the fused cells grown in the 24-well plate was confirmed by ELISA, only fused cells having an absorbance of more than 1 were selected and then transferred to a 6-well culture flask for incubation, and a supernatant was collected after centrifugation and subjected to ELISA, followed by tertiary screening. The fused cells selected based on the tertiary screening were then transferred to a 75T/C culture flask and cultured, absorbance was confirmed by ELISA, and then only fused cells having an absorbance of more than 1 were selected.

### (4) Epitope mapping of monoclonal antibody against IgE pep-40 protein

Epitope mapping was performed using a peptide used in antibody production. The peptide used for mapping was a peptide having a 12, 13 or 15-amino-acid sequence, which is a part of the IgE pep-40 protein used in conventional antigen production (Table 3).

An antibody binding to a Pep-6 peptide among the above-described peptides was found, and Pep-6 was determined as an epitope of a novel antigen (IgE pep-40), which was previously unknown.

**[Table 3]**

| Peptide type | Amino acid sequence | SEQ ID NO: |
|---|---|---|
| Pep-1 | LEDGQVMDVDLST | 1 |
| Pep-4 | EVTRAEWEQKDE | 2 |
| **Pep-6** | **SRASGKPVNHSTR** | **3** |

Example 2. Manufacture of kit for diagnosing or analyzing a prognosis of allergic disease

### (1) Allergen isolation

Ether was added to immerse a TSM allergen source, and then well mixed with raw materials listed in Table 4 below, followed by incubation at room temperature for 5 minutes.

**[Table 4]**

| Name of raw material | Specification | Amount | Name of raw material | Specification | Amount |
|---|---|---|---|---|---|
| Anti-Ige | Company standard | 0.1 µg | Cat | Company standard | 0.5 µg |
| *Dermatophagoides pteronyssinus* | Company standard | 0.5 µg | Alder | Company standard | 0.3 µg |
| *Dermatophagoides farinae* | Company standard | 0.5 µg | Birch | Company standard | 0.5 µg |
| *Lolium perenne* L. | Company standard | 0.5 µg | Soybean | Company standard | 0.5 µg |
| Oak | Company standard | 0.5 µg | Walnut | Company standard | 0.5 µg |
| Mugwort | Company standard | 0.5 µg | Cod | Company standard | 0.5 µg |
| *Humulus japonicus* | Company standard | 0.5 µg | *Dactylis glomerata* | Company standard | 0.5 µg |
| Garlic | Company standard | 0.5 µg | Milk | Company standard | 0.5 µg |
| Pork | Company standard | 0.3 µg | Peach | Company standard | 0.5 µg |
| Tuna | Company standard | 0.5 µg | Potato | Company standard | 0.5 µg |
| Alternaria alternate | Company standard | 0.5 µg | *Ambrosia artemisiifolia* L. | Company standard | 0.5 µg |
| Dog | Company standard | 0.5 µg | Egg white | Company standard | 0.5 µg |
| Sesame | Company standard | 0.5 µg | Wheat | Company standard | 0.5 µg |
| *Cladosporium herbarum* | Company standard | 0.5 µg | Crab | Company standard | 0.3 µg |
| *Aspergillus fumigateus* | Company standard | 0.3 µg | Barley | Company standard | 0.5 µg |
| Peanut | Company standard | 0.2 µg | Reed | Company standard | 0.5 µg |
| Beef | Company standard | 0.5 µg | Cockroach | Company standard | 0.5 µg |
| *Anthoxanthum odoratum* L. | Company standard | 0.5 µg | *Phleum pratense* | Company standard | 0.3 µg |
| Shrimp | Company standard | 0.5 µg | Buckwheat | Company standard | 0.5 µg |
| Apple | Company standard | 0.5 µg | House dust | Company standard | 0.5 µg |

Subsequently, ether was carefully poured out and fresh ether was added again, which was repeated three times for defatting, thereby preparing a sample. Afterward, i) each sample was mixed with PBS (pH 8.0) at 1:5 (v/v), extracted using a Polytron Homogenizer on ice, and incubated at 4 °C for 24 hours, or ii) a COCA's solution was added to the sample at a volume 5-fold higher than the sample, and then incubated while rotating at 4 °C for 24 hours.

Subsequently, a supernatant was separated by centrifugation at 4 °C and 15,000 rpm for 30 minutes, and then filtered through Whatman paper. Afterward, the sample was dialyzed using 0.1 x PBS (pH 7.5) for 24 to 48 hours. The dialyzed sample was air-dried as is in a refrigerator to decrease a volume approximately 1/10. After centrifugation at 4 °C and 15,000 rpm for 1 hour, the resulting product was filtered through a 45-µm syringe filter. A protein was then quantified using a Bradford assay, and then freeze-dried.

### (2) Preparation of antibody binding to Pep-6

### 1) Confirmation of antibody titer

An antibody binding to the Pep-6 peptide was found, a monoclonal antibody cell line using the same was established, and then an antibody titer confirming test was carried out using a cell culture solution. Here, as a Pep-6 carrier, a KLH protein was used.

**[Table 5]**

| Clone | Pep6-KLH | Human IgE | KLH |
|---|---|---|---|
| **8-7_100cell** | **1.5540** | **0.4070** | **0.0660** |
| 59-3_500cell | 1.3840 | 0.1000 | 0.0670 |
| 67-2_500cell | 1.5000 | 0.2470 | 0.0670 |
| **140-13_500cell** | **1.5090** | **0.3320** | **0.0650** |
| 213-1_100cell | 1.3890 | 0.1310 | 0.0660 |
| 285-1_100cell | 1.5030 | 0.2150 | 0.0630 |
| **302-2_100cell** | **1.4480** | **0.3030** | **0.0670** |
| 370-7_500cell | 0.9040 | 0.0620 | 0.0640 |
| 373-3_500cell | 1.5030 | 0.1770 | 0.0670 |
| 405-1_100cell | 1.4370 | 0.2020 | 0.0640 |
| H5-14_500cell | 1.4040 | 0.2130 | 0.0630 |
| H8-1_500cell | 1.3990 | 0.0830 | 0.0640 |
| 563-5 (separating) | 1.4420 | 0.1210 | 0.0640 |
| Blank | 0.0610 | 0.0580 | 0.0580 |

| | | | |
|---|---|---|---|
| *93Plate coating: Pep-6-KLH, Human IgE, KLH Blank: negative control to which cell culture solution was not added | | | |

As a result, the result shown in Table 5 was able to be confirmed, and it can be confirmed that 8-7_100cell, 140-13_500cell, and 302-2_100cell did not bind to KLH, but had binding strength to a recombinant IgE whole protein and high binding strength to pep-6-KLH.

### 2) Comparison of performance with commercially available antibody

A test was performed to compare the performance of the 8-7_100cell, 140-13_500cell and 302-2_100cell, which were confirmed to have the highest binding strength to Pep-6-KLH from the confirmation of an antibody titer, with a commercially available anti-IgE antibody (Table 6).

**[Table 6]**

| Clone | Pep6-KLH | Human IgE | KLH |
|---|---|---|---|
| **8-7_100cell** | **1.7200** | **0.4420** | **0.0820** |
| 59-3_500cell | 1.5030 | 0.1440 | 0.0840 |
| 67-2_500cell | 1.5930 | 0.2330 | 0.0870 |
| **140-13_500cell** | **1.6540** | **0.3670** | **0.0850** |
| 213-1_100cell | 1.5020 | 0.1410 | 0.0810 |
| 285-1_100cell | 1.5730 | 0.2240 | 0.0770 |
| **302-2_100cell** | **1.5370** | **0.3450** | **0.0740** |
| 370-7_500cell | 1.0410 | 0.0820 | 0.0790 |
| 373-3_500cell | 1.6430 | 0.2120 | 0.0880 |
| 405-1_100cell | 1.5620 | 0.2220 | 0.1090 |
| H5-14_500cell | 1.5510 | 0.2590 | 0.0850 |
| H8-1_500cell | 1.4720 | 0.1060 | 0.0830 |
| 563-5 (separating) | 1.5450 | 0.1530 | 0.0820 |
| Anti-IgE (2 µg/ml, commercially available) | 0.0800 | 0.4940 | 0.0760 |
| Blank | 0.0800 | 0.0740 | 0.0770 |

As a result, as shown in Table 6, it can be seen that three types of antibodies (8-7_100cell, 140-13_500cell and 302-2_100cell) are antibodies with binding sites different from that of the commercially available antibody, but have similar binding strengths to recombinant IgE, and it was determined that the three types of antibodies (8-7_100cell, 140-13_500cell and 302-2_100cell) can be used to manufacture a kit.

### 3) Confirmation of binding strength of selected antibody to native IgE

As an antibody used to manufacture a kit, three types of antibodies (8-7_100cell, 140-13_500cell and 302-2_100cell) similar to a commercially available product in terms of binding strength to recombinant IgE were selected. In addition, to confirm the degree of binding strength of the three types of antibodies for native IgE present in blood, an ELISA test was performed by the following method.

2 µg/mL of the commercially available antibodies against human IgE were reacted at 100 µL per well and 4 °C for 18 hours to coat the wells. The next day, the antibodies were reacted (blocking) using a blocking buffer at room temperature for 1 hour. During blocking, the selected three types of antibodies were biotinylated according to the protocol of a commercially available biotinylation kit. Afterward, 10 µL of a recombinant IgE protein and the serum of a patient diagnosed as having an allergy were dispensed into wells, and reacted at room temperature for 1 hour. After the reaction was completed the plate was washed with PBST, and then, HRP-conjugated streptavidin was diluted 1:5000 and dispensed at 100 µL per well to allow a reaction at room temperature for 1 hour. Subsequently, the plate was washed with PBST, and reacted for 15 minutes after a TMB solution was dispensed, followed by termination of the reaction using a reaction stop solution. Absorbance was measured at 450/620 nm to confirm the degree of binding of the selected antibodies to recombinant IgE and native IgE.

**[Table 7]**

| | Recombinant | | | Serum Y | | | Serum L | | |
|---|---|---|---|---|---|---|---|---|---|
| Concentration (10 µg/ml) | 8-7-100cell | 140-13-500cell | 302-2-100cell | 8-7-100cell | 140-13-500cell | 302-2-100cell | 8-7-100cell | 140-13-500cell | 302-2-100cell |
| 10 | 0.615 | 0.61 | 0.612 | 1.374 | 1.375 | 1.374 | 1.43 | 1.431 | 1.430 |
| 5 | 0.504 | 0.507 | 0.505 | 1.077 | 1.082 | 1.079 | 1.133 | 1.138 | 1.135 |
| 2.5 | 0.416 | 0.403 | 0.409 | 0.847 | 0.859 | 0.853 | 0.903 | 0.915 | 0.909 |
| 1.25 | 0.317 | 0.307 | 0.312 | 0.616 | 0.614 | 0.615 | 0.672 | 0.67 | 0.671 |
| 0.625 | 0.224 | 0.214 | 0.219 | 0.384 | 0.375 | 0.379 | 0.44 | 0.431 | 0.435 |
| 0.3125 | 0.132 | 0.129 | 0.130 | 0.185 | 0.173 | 0.179 | 0.241 | 0.229 | 0.235 |
| Blank | 0.041 | 0.047 | 0.055 | 0.04 | 0.039 | 0.038 | 0.038 | 0.038 | 0.038 |

As a result of the test, as shown in Table 7, it can be confirmed that the selected three types of antibodies bound to recombinant IgE and native IgE, and it was determined that all of the antibodies can be used to manufacture a kit.

### (3) Preparation of gold conjugate

Colloidal gold particles having a diameter of 40 to 60 nm were prepared using chloroauric acid and a reducing agent. Tertiary distilled water was added to the colloidal gold particles so that an OD value at 520 nm was 1.0 ± 1.0. 5 to 15 µg of the monoclonal anti-human IgE antibody prepared in Example 1-2 was added to 1 mL of the gold solution, and then shaken at room temperature for 1 hour. After the reaction was completed, the gold particles were washed, quantified using a spectrophotometer at 520 nm, and diluted so that an OD value became 5.0 ± 1.0, followed by addition of a preservative.

### (4) Manufacture of kit and device

The allergen prepared in Example 1-(1) was diluted to 5 mg/mL using a borate buffer, anti-mouse IgG was diluted to 2 mg/mL, and the resulting diluents were dispensed at test line and control line positions using an automatic dispenser to coat a membrane. Afterward, the coated membrane was put on an inspection stage, and whether each coating solution is normally dispensed was visually inspected. The normally-dispensed membrane was dried at a constant temperature of 37±2 °C for 1 hour or more, and sealed before assembly, followed by storage in a dehumidified state.

The gold conjugated prepared in Example 1-(3) was mixed with a stabilizer to be uniformly absorbed into glass fibers. Here, as 19.63 µL of the gold conjugate per 1.1 cm x 0.4 cm of glass fibers was used, a monoclonal anti-human IgE antibody gold conjugate was added to one device at a mass of 14.59 ± 1.06 µg based on a stock solution. The glass fibers dried at 37±2 °C for 1 hour were sealed before assembly, and stored in a dehumidified state.

A conjugate pad and a specimen pad were sequentially attached so as to overlap at the bottom of the coated membrane, and an absorption pad was attached at the top of the coated membrane. The resulting pad was cut to a specification of (45 ± 5.2) mm x (4.4 ± 1.5) mm using a special cutting machine, and stored before assembly of a device after water-proof packaging. Afterward, the cut strip was put on a lower plastic device, covered with an upper plastic device, and then put into a silver foil pouch with a dried silica gel, followed by sealed packaging.

### [Experimental Example]

### Confirmation of limit of detection

To confirm the limit of detection of the kit manufactured in Example 2, in an immuno-CAP test, specimens testing positive (3.5 IU/mL or more) were diluted 0, 2-, 4-, 8-, 16- and 32-fold, and the specimen for each allergen was tested three times according to a standard test method and then judged according to judgment criteria. The results are shown in Table 8 below.

**[Table 8]**

| **Allergen** | **Strip No.** | **Specimen No.** | **Test method (judgment)** | **1X** | **2X** | **4X** | **8X** | **16X** | **32X** |
|---|---|---|---|---|---|---|---|---|---|
| Total IgE | | **hA-SP0403** | Immuno_CAP (IU/ml) | 213.0 | **106.50** | 53.25 | 26.63 | 13.31 | 6.66 |
| | | | SSmarTEST | positive | **positive** | negative | negative | negative | negative |
| Birch | 1-2 | **hA-SP0221** | Immuno_CAP (IU/ml) | 73.36 | 36.68 | 18.34 | 9.17 | **4.59** | 2.29 |
| | | | SSmarTEST | positive | positive | positive | positive | **positive** | negative |
| *Dactylis glomerata* | 1-3 | **hA-SP0261** | Immuno_CAP (IU/ml) | 8.70 | **4.35** | 2.18 | 1.09 | 0.54 | 0.27 |
| | | | SSmarTEST | positive | **positive** | negative | negative | negative | negative |
| *Anthoxanthum odoratum* L. | 1-4 | **hA-SP0173** | Immuno_CAP (IU/ml) | 9.04 | **4.52** | 2.26 | 1.13 | 0.57 | 0.28 |
| | | | SSmarTEST | positive | **positive** | negative | negative | negative | negative |
| *Lolium perenne* L. | 1-5 | **hA-SP0032** | Immuno_CAP (IU/ml) | 31.40 | 15.70 | 7.85 | **3.93** | 1.96 | 0.98 |
| | | | SSmarTEST | positive | positive | positive | **positive** | negative | negative |
| Oak | 1-6 | **hA-SP0042** | Immuno_CAP (IU/ml) | **4.07** | 2.04 | 1.02 | 0.51 | 0.25 | 0.13 |
| | | | SSmarTEST | **positive** | negative | negative | negative | negative | negative |
| Alder | 1-7 | **hA-SP0213** | Immuno_CAP (IU/ml) | **4.21** | 2.11 | 1.05 | 0.53 | 0.26 | 0.13 |
| | | | SSmarTES | **positi ve** | negative | negative | negative | negative | negative |
| *Ambrosia artemisiifolia* L. | 1-8 | **hA-SP0301** | Immuno_CAP (IU/ml) | 16.30 | 8.15 | **4.08** | 2.04 | 1.02 | 0.51 |
| | | | SSmarTEST | positive | positive | **positive** | negative | negative | negative |
| Mugwort | 1-9 | **hA-SP0052** | Immuno_CAP (IU/ml) | **4.38** | 2.19 | 1.10 | 0.55 | 0.27 | 0.14 |
| | | | SSmarTEST | **positive** | negative | negative | negative | negative | negative |
| D.P. | 1-10 | **hA-SP0014** | Immuno_CAP (IU/ml) | 57.05 | 28.53 | 14.26 | 7.13 | **3.57** | 1.78 |
| | | | SSmarTEST | positive | positive | positive | positive | **positive** | negative |
| Apple | 2-1 | **hASP0192** | Immuno_CAP (IU/ml) | **4.42** | 2.21 | 1.11 | 0.55 | 0.28 | 0.14 |
| | | | SSmarTEST | **positive** | negative | negative | negative | negative | negative |
| Buckwheat | 2-2 | **hA-SP0371** | Immuno_CAP (IU) | **5.91** | 2.96 | 1.48 | 0.74 | 0.37 | 0.18 |
| | | | SSmarTEST | **positive** | negative | negative | negative | negative | negative |
| Cockroach | 2-3 | **hA-SP0381** | Immuno_CAP (IU/ml) | **4.35** | 2.18 | 1.09 | 0.54 | 0.27 | 0.14 |
| | | | SSmarTEST | **positive** | negative | negative | negative | negative | negative |
| *Aspergillus fumigateus* | 2-4 | **hA-SP0142** | Immuno_CAP (IU/ml) | 8.15 | **4.08** | 2.04 | 1.02 | 0.51 | 0.25 |
| | | | SSmarTEST | positive | **positive** | negative | negative | negative | negative |
| *Cladosporium herbarum* | 2-5 | **hA-SP0133** | Immuno_CAP (IU/ml) | **4.92** | 2.46 | 1.23 | 0.62 | 0.31 | 0.15 |
| | | | SSmarTEST | **positive** | negative | negative | negative | negative | negative |
| *Alternaria alternate* | 2-6 | **hA-SP0102** | Immuno_CAP (IU/ml) | 9.28 | **4.64** | 2.32 | 1.16 | 0.58 | 0.29 |
| | | | SSmarTEST | positive | **positive** | negative | negative | negative | negative |
| *Humulus japonicus* | 2-7 | **hA-SP0062** | Immuno_CAP (IU/ml) | 7.45 | **3.73** | 1.86 | 0.93 | 0.47 | 0.23 |
| | | | SSmarTEST | positive | **positive** | negative | negative | negative | negative |
| Reed | 2-8 | **hA-SP0352** | Immuno_CAP (IU/ml) | **4.37** | 2.19 | 1.09 | 0.55 | 0.27 | 0.14 |
| | | | SSmarTEST | **positive** | negative | negative | negative | negative | negative |
| *Phleum pratense* | 2-9 | **hA-SP0361** | Immuno_CAP (IU/ml) | **3.90** | 1.95 | 0.98 | 0.49 | 0.24 | 0.12 |
| | | | SSmarTEST | **positive** | negative | negative | negative | negative | negative |
| D.F. | 2-10 | **hA-SP0022** | Immuno_CAP (IU/ml) | 60.20 | 30.10 | 15.05 | 7.53 | **3.76** | 1.88 |
| | | | SSmarTEST | positive | positive | positive | positive | **positive** | negative |
| Wheat | 3-1 | **hA-SP0322** | Immuno_CAP (IU/ml) | 47.23 | 23.62 | 11.81 | **5.90** | 2.95 | 1.48 |
| | | | SSmarTEST | positive | positive | positive | **positive** | negative | negative |
| Garlic | 3-2 | **hA-SP0073** | Immuno_CAP (IU/ml) | 7.57 | **3.79** | 1.89 | 0.95 | 0.47 | 0.24 |
| | | | SSmarTEST | positive | **positive** | negative | negative | negative | negative |
| Milk | 3-3 | **hA-SP0271** | Immuno_CAP (IU/ml) | 32.07 | 16.04 | 8.02 | **4.01** | 2.00 | 1.00 |
| | | | SSmarTEST | positive | positive | positive | **positive** | negative | negati e |
| Sesame | 3-4 | **hA-SP0122** | Immuno_CAP (IU/ml) | **4.06** | 2.03 | 1.02 | 0.51 | 0.25 | 0.13 |
| | | | SSmarTEST | **positive** | negative | negative | negative | negative | negative |
| Peanut | 3-5 | **hA-SP0153** | Immuno_CAP (IU/ml) | 20.66 | 10.33 | **5.17** | 2.58 | 1.29 | 0.65 |
| | | | SSmarTEST | positive | positive | **positive** | negative | negative | negative |
| Potato | 3-6 | **hA-SP0292** | Immuno_CAP (IU/ml) | **3.81** | 1.91 | 0.95 | 0.48 | 0.24 | 0.12 |
| | | | SSmarTEST | **positive** | negative | negative | negative | negative | negative |
| Soybean | 3-7 | **hA-SP0231** | Immuno_CAP (IU/ml) | 30.60 | 15.30 | 7.65 | **3.83** | 1.91 | 0.96 |
| | | | SSmarTEST | positive | positive | positive | **positive** | negative | negative |
| Egg white | 3-8 | **hA-SP0313** | Immuno_CAP (IU/ml) | 29.15 | 14.58 | 7.29 | **3.64** | 1.82 | 0.91 |
| | | | SSmarTEST | positive | positive | positive | **positive** | negative | negative |
| Cat | 3-9 | **hA-SP0202** | Immuno_CAP (IU/ml) | 76.83 | 38.42 | 19.21 | 9.60 | **4.80** | 2.40 |
| | | | SSmarTEST | positive | positive | positive | positive | **positive** | negative |
| House dust | 3-10 | **hA-SP0392** | Immuno_CAP (IU/ml) | 15.46 | 7.73 | **3.87** | 1.93 | 0.97 | 0.48 |
| | | | SSmarTEST | positive | positive | **positive** | negative | negative | negative |
| Peach | 4-1 | **hA-SP0281** | Immuno_CAP (IU/ml) | 8.92 | **4.46** | 2.23 | 1.12 | 0.56 | 0.28 |
| | | | SSmarTEST | positive | **positive** | negative | negative | negative | negative |
| Barley | 4-2 | **hA-SP0341** | Immuno_CAP (IU/ml) | **4.02** | 2.01 | 1.01 | 0.50 | 0.25 | 0.13 |
| | | | SSmarTEST | **positive** | negative | negative | negative | negative | negative |
| Cod | 4-3 | **hA-SP0251** | Immuno_CAP (IU/ml) | **4.50** | 2.25 | 1.13 | 0.56 | 0.28 | 0.14 |
| | | | SSmarTEST | **positive** | negative | negative | negative | negative | negative |
| Pork | 4-4 | **hA-SP0081** | Immuno_CAP (IU/ml) | 7.37 | **3.69** | 1.84 | 0.92 | 0.46 | 0.23 |
| | | | SSmarTEST | positive | **positive** | negative | negative | negative | negative |
| Walnut | 4-5 | **hA-SP0241** | Immuno_CAP (IU/ml) | 72.90 | 36.45 | 18.23 | 9.11 | **4.56** | 2.28 |
| | | | SSmarTEST | positive | positive | positive | positive | **positive** | negative |
| Crab | 4-6 | **hA-SP0331** | Immuno_CAP (IU/ml) | 8.60 | **4.30** | 2.15 | 1.08 | 0.54 | 0.27 |
| | | | SSmarTEST | positive | **positive** | negative | negative | negative | negative |
| Tuna | 4-7 | **hA-SP0091** | Immuno_CAP (IU/ml) | **3.97** | 1.99 | 0.99 | 0.50 | 0.25 | 0.12 |
| | | | SSmarTEST | **positive** | negative | negative | negative | negative | negative |
| Beef | 4-8 | **hA-SP0162** | Immuno_CAP | **5.89** | 2.95 | 1.47 | 0.74 | 0.37 | 0.18 |
| | | | SSmarTEST | **positive** | negative | negative | negative | negative | negative |
| Shrimp | 4-9 | **hA-SP0183** | Immuno_CAP (IU/ml) | **3.90** | 1.95 | 0.98 | 0.49 | 0.24 | 0.12 |
| | | | SSmarTEST | **positive** | negative | negative | negative | negative | negative |
| Dog | 4-10 | **hA-SP0112** | Immuno_CAP (IU/ml) | 58.47 | 29.24 | 14.62 | 7.31 | **3.65** | 1.83 |
| | | | SSmarTEST | positive | positive | positive | positive | **positive** | negati e |

As a result, as shown in Table 8, a plurality of specimens per allergen were diluted in 5 steps, and each diluted specimen was tested three times. Among all tested specimens, in a specimen testing positive in all three tests, the lowest Immuno-Cap quantitative value was set as the limit of detection of each allergen. That is, since the limit of detection of the kit according to one embodiment was determined to be positive when the quantitative value was 2 to 8 ng, but not all of the specimens having a quantitative value of 1.68 to 8.39 ng were determined to be positive, the quantitative value of 8.4 ng or more based on EIA class was set as the limit of detection.

### Confirmation of precision and accuracy

To determine the accuracy for an allergen between (1) testers, (2) test days, (3) lots, and (4) on the same test day and between test sites of the kit manufactured in Example 2, a test was performed according to a standard test method. A negative reference material and a positive reference material for each allergen were prepared to perform a test by the following test method, and the results are shown in Table 9 below.

### (1) Between testers

Three different testers tested the kit manufactured in Example 2 using a negative reference material and a positive reference material, and the results were analyzed.

### (2) Between test days

One tester tested the kit manufactured in Example 2 using a negative reference material and a positive reference material every day for 5 days, and the results were analyzed.

### (3) Between lots

One tester tested the three kits manufactured in Example 2 using a negative reference material and a positive reference material, and the results were analyzed.

### (4) On the same test day (between test sites)

One tester tested the kit manufactured in Example 2 at three different places (a laboratory, a QC room, etc.) using a negative reference material and a positive reference material, and the results were analyzed.

**[Table 9]**

| Test item | Test classification | Test results | | |
|---|---|---|---|---|
| | | Negative reference material | Positive reference material | |
| | | | Low positive | High positive |
| Between testers | Tester 1 | 3/3* | 3/3** | 3/3** |
| | Tester 2 | 3/3* | 3/3** | 3/3** |
| | Tester 3 | 3/3* | 3/3** | 3/3** |
| Between test days | 2017.06.19 | 3/3* | 3/3** | 3/3** |
| | 2017.06.20 | 3/3* | 3/3** | 3/3** |
| | 2017.06.21 | 3/3* | 3/3** | 3/3** |
| | 2017.06.22 | 3/3* | 3/3** | 3/3** |
| | 2017.06.23 | 3/3* | 3/3** | 3/3** |
| Between lots | huAL1706-001 | 3/3* | 3/3** | 3/3** |
| | huAL1706-002 | 3/3* | 3/3** | 3/3** |
| | huAL1706-003 | 3/3* | 3/3** | 3/3** |
| On the same test day (between test sites) | QC room, Genome Medicine Research Institute | 3/3* | 3/3** | 3/3** |
| | Metabolite Division Laboratory | 3/3* | 3/3** | 3/3** |
| | QC Division Laboratory | 3/3* | 3/3** | 3/3** |
| Same person | Tester 1 | 36/36* | 36/36** | 36/36** |
| Abb.: *, tested negative, kit number/test kit number, **, tested positive, kit number/test kit number | | | | |

As a result, as shown in Table 9, it was confirmed that the device of Example 2 shows the same test results which are negative in the test using the negative reference material and positive in the test using the positive reference material regardless of the same person, a tester, a test day, lot and a test site.

### Confirmation of specificity

### Confirmation of false positive or false negative

To measure an interference effect by a substance present in a specimen, materials listed in Table 10 below were added to a negative reference material and a positive reference material to test for false-positives or false-negatives, and the results are shown in Table 11 below.

**[Table 10]**

| Test material | Concentration |
|---|---|
| Human albumin | 20 g/dL |
| Sodium citrate | 500 mg/dL |
| Cvanocobalamin (vitamin B12) | 1 mg/dL |
| Bilirubin | 20 mg/dL |
| EDTA | 800 mg/dL |
| Glucose | 10 g/dL |
| Ascorbic acid | 521 mg/dL |
| Hemoglobin | 250 mg/dL |

**[Table 11]**

| | Primary test | | Secondary test | | Tertiary test | |
|---|---|---|---|---|---|---|
| | Negative reference material | Positive reference material | Negative reference material | Positive reference material | Negative reference material | Positive reference material |
| Human albumin | negative | positive | negative | positive | negative | positive |
| Sodium citrate | negative | positive | negative | positive | negative | positive |
| Cvanocobalamin | negative | positive | negative | positive | negative | positive |
| Bilirubin | negative | positive | negative | positive | negative | positive |
| EDTA | negative | positive | negative | positive | negative | positive |
| Glucose | negative | positive | negative | positive | negative | positive |
| Ascorbic acid | negative | positive | negative | positive | negative | positive |
| Hemoglobin | negative | positive | negative | positive | negative | positive |

Here, the false-positive means that a negative reference material is determined to be positive by an interfering substance, and the false-negative means that a positive reference material including an interfacing substance is determined to be negative due to the interfering substance.

As a result, as shown in Table 11, it was able to be confirmed that the 8 types of interfering substances did not have any effect on the kit according to the present invention.

### Confirmation whether crosslinking reaction occurred

In addition, to confirm the presence or absence of crosslinking caused by non-specific bonds between antibodies used in a test and the immunoglobulin isotypes that can be present in a specimen, 3 mg/mL each of IgA, IgM, IgG and IgD was added to a negative reference material to perform a test according to a standard test method, and judged according to judgment criteria.

As a result, all of the negative reference materials to which IgA, IgM, IgG and IgD were added were tested negative, confirming that there was no interference response.

### UniCAP test comparison with commercially available products

An allergy kit was manufactured according to Example 2 using 8-7_100 cell of the three types of antibodies prepared and selected according to Example 1-2 to perform a test of confirming a match rate with a commercially available kit (LG, Alloscan) for 13 types of allergens (*Dermatophagoides pteronyssinus* (*D. pteronyssinus*), *D. farinae,* cat, house dust mite, dog, birch, *Alternaria,* peach, apple, *Lolium perenne* L., *Anthoxanthum odoratum* L., *Dactylis glomerata, Phleum pratense,* oak, egg white and potato) with high incidence among 39 types of allergens. The test used 160 specimens testing positive for one or more allergens among the 13 types of allergens, and specimens showing inconsistency were subjected to a uniCAP test considered as a third test method as well as a standard test method of an allergic test to judge the results. The test using a commercially available kit and the uniCAP test were conducted by a contract research organization, and self-developed kits were tested by two inspectors (Table 12).

**[Table 12]**

| Represent result value as negative or positive | | | | | | |
|---|---|---|---|---|---|---|
| | | | | | | |
| No. | Allergen | Name of patient | LG mast | SLSbio | uniCAP | SLS:uniCAP |
| 1 | DP | Specimen 1 | + | + | + | Matched |
| 2 | | Specimen2 | + | + | + | Matched |
| 3 | | Specimen 3 | + | + | + | Matched |
| 4 | | Specimen 4 | + | + | + | Matched |
| 5 | | Specimen 5 | + | - | - | Matched |
| 6 | | Specimen 6 | + | - | + | Mismatched |
| 7 | | Specimen 7 | + | - | - | Matched |
| 8 | | Specimen 8 | + | - | - | Matched |
| 9 | | Specimen 9 | + | - | - | Matched |
| 10 | | Specimen 10 | + | + | + | Matched |
| 11 | DF | Specimen 11 | + | + | + | Matched |
| 12 | | Specimen 12 | + | + | + | Matched |
| 13 | | Specimen 13 | + | + | + | Matched |
| 14 | | Specimen 14 | + | - | - | Matched |
| 15 | | Specimen 15 | + | - | - | Matched |
| 16 | | Specimen 16 | + | + | + | Matched |
| 17 | | Specimen 17 | + | - | - | Matched |
| 18 | | Specimen 18 | + | - | - | Matched |
| 19 | | Specimen 19 | + | + | + | Matched |
| 20 | | Specimen 20 | + | + | + | Matched |
| 21 | | Specimen 21 | + | + | + | Matched |
| 22 | | Specimen 22 | + | - | - | Matched |
| 23 | | Specimen 23 | + | + | + | Matched |
| 24 | Cat | Specimen 24 | + | - | - | Matched |
| 25 | | **Specimen 25** | + | + | - | **Mismatched** |
| 26 | | Specimen 26 | + | - | - | Matched |
| 27 | | Specimen 27 | + | + | + | Matched |
| 28 | | **Specimen 28** | + | + | - | **Mismatched** |
| 29 | | Specimen 29 | + | - | - | Matched |
| 30 | | Specimen 30 | + | - | - | Matched |
| 31 | | Specimen 31 | + | - | - | Matched |
| 32 | | Specimen 32 | + | + | + | Matched |
| 33 | | Specimen 33 | + | - | - | Matched |
| 34 | | Specimen 34 | + | + | + | Matched |
| 35 | | Specimen 35 | + | + | + | Matched |
| 36 | | **Specimen 36** | + | - | + | **Mismatched** |
| 37 | House dust | Specimen 37 | + | - | - | Matched |
| 38 | | Specimen 38 | + | - | - | Matched |
| 39 | | Specimen 39 | + | - | - | Matched |
| 40 | | Specimen 40 | + | - | - | Matched |
| 41 | | Specimen 41 | + | - | - | Matched |
| 42 | | Specimen 42 | + | - | - | Matched |
| 43 | | Specimen 43 | + | - | - | Matched |
| 44 | | Specimen 44 | + | - | - | Matched |
| 45 | | Specimen 45 | + | - | - | Matched |
| 46 | Dog | Specimen 46 | + | - | - | Matched |
| 47 | | Specimen 47 | + | - | - | Matched |
| 48 | | Specimen 48 | + | - | - | Matched |
| 49 | | Specimen 49 | + | - | - | Matched |
| 50 | | Specimen 50 | + | - | - | Matched |
| 51 | | Specimen 51 | + | - | - | Matched |
| 52 | | Specimen 52 | + | + | + | Matched |
| 53 | | Specimen 53 | + | - | - | Matched |
| 54 | | Specimen 54 | + | - | - | Matched |
| 55 | | Specimen 55 | + | + | + | Matched |
| 56 | | Specimen 56 | + | + | + | Matched |
| 57 | | **Specimen 57** | + | - | + | **Mismatched** |
| 58 | | Specimen 58 | + | + | + | Matched |
| 59 | Birch | Specimen 59 | + | + | + | Matched |
| 60 | | Specimen 60 | + | - | - | Matched |
| 61 | | **Specimen 61** | + | - | + | **Mismatched** |
| 62 | | **Specimen 62** | + | - | + | **Mismatched** |
| 63 | | Specimen 63 | + | + | + | Matched |
| 64 | | **Specimen 64** | + | - | + | **Mismatched** |
| 65 | | Specimen 65 | + | + | + | Matched |
| 66 | Alternaria | **Specimen 66** | + | - | + | **Mismatched** |
| 67 | | **Specimen 67** | + | - | + | **Mismatched** |
| 68 | | **Specimen 68** | + | - | + | **Mismatched** |
| 69 | | **Specimen 69** | + | - | + | **Mismatched** |
| 70 | | **Specimen 70** | + | - | + | **Mismatched** |
| 71 | Peach | Specimen 71 | + | - | - | Matched |
| 72 | | Specimen 72 | + | - | - | Matched |
| 73 | | Specimen 73 | + | - | - | Matched |
| 74 | Apple | **Specimen 74** | + | - | + | **Mismatched** |
| 75 | | **Specimen 75** | + | - | + | **Mismatched** |
| 76 | *Lolium perenne* L. | Specimen 76 | + | - | - | Matched |
| 77 | Oak | Specimen 77 | + | - | - | Matched |
| 78 | Egg white | Specimen 78 | + | + | + | Matched |
| 79 | | **Specimen 79** | + | + | - | **Mismatched** |
| 80 | | Specimen 80 | + | + | + | Matched |
| 81 | | Specimen 81 | + | - | - | Matched |
| 82 | | Specimen 82 | + | + | + | Matched |

As a result, as shown in Table 12, among the 160 specimens, inconsistency was found in 52 specimens, and among the inconsistent specimens, as a result of the uniCAP test, 36 specimens were consistent with the test using the self-developed kit using an antibody, and 16 specimens were consistent with the test using a commercially available kit.

The result showed that the allergic test kit using an antibody according to Example 2 has a non-specific response to an antibody or excellent binding strength with an allergen, indicating a high match rate.

It would be understood by those of ordinary skill in the art that the above descriptions of the present invention are exemplary, and the example embodiments disclosed herein can be easily modified into other specific forms without changing the technical spirit or essential features of the present invention. Therefore, it should be interpreted that the example embodiments described above are exemplary in all aspects, and are not limitative.

## Claims

1. A peptide consisting of the amino acid sequence of SEQ ID No: 3, which is an epitope of immunoglobulin E (IgE).

2. An anti-immunoglobulin E (IgE) antibody binding to the peptide of claim 1.

3. A kit for analyzing immunoglobulin E (IgE) in a sample, the kit comprising the antibody of claim 2.

4. The kit of claim 3, further comprising a carrier or a diluent.

5. The kit of claim 3, wherein the sample is a biological sample which is isolated from a subject after contacting the subject with an allergen.

6. The kit of claim 5, wherein the sample is isolated 10 to 40 minutes after contact.

7. The kit of claim 3, comprising an allergen and a membrane coated with the antibody.

8. The kit of claim 7, wherein the allergen is any one or more selected from the group consisting of a house dust mite, *Lolium perenne* L., oak, mugwort, *Humulus japonicus,* garlic, pork, tuna, fungus, a dog, sesame, peanut, beef, *Anthoxanthum odoratum* L., shrimp, apple, a cat, alder, birch, soybean, walnut, cod, *Dactylis glomerata,* milk, peach, potato, *Ambrosia artemisiifolia* L., egg white, wheat, crab, barley, reed, a cockroach, buckwheat and house dust.

9. The kit of claim 7, wherein the membrane is selected from the group consisting of nitrocellulose, nylon, polyvinylidene fluoride (PVDF), glass and plastic.

10. The kit of claim 3, which is for immunochromatography.

11. A device for diagnosing or predicting a prognosis of an allergy, which comprises:
the kit of claim 3; and a specimen loading part.

12. A method of diagnosing an allergic disease, comprising:
collecting blood of a subject and mixing it with a specimen diluent; and
dropping the dilution into the specimen loading part to perform a reaction.

13. The method of claim 12, wherein the reaction is performed for 10 to 40 minutes.

14. The method of claim 12, wherein a volume of the blood is 0.5 to 2 mL.

15. The method of claim 12, wherein the blood is plasma or serum.

16. The method of claim 15, wherein a volume of the plasma or serum is 0.1 to 1 mL.


<110>    SLSBio

<120>    A NOVEL EPITOPE OF IMMUNOGLOBULIN E, ANTIBODY BINDING THERETO AND
         A KIT COMPRISING ABOVE ANTIBODY FOR ANALYSIS OF IMMUNOGLOBULIN E

<130>    PX058494PCT

<150>    KR 10/2018/0040605
<151>    2018-04-06

<160>    3

<170>    KoPatentIn 3.0

<210>    1
<211>    13
<212>    PRT
<213>    Artificial Sequence

<220>
<223>    Pep-1


<400>    1
Leu Glu Asp Gly Gln Val Met Asp Val Asp Leu Ser Thr
  1               5                  10


<210>    2
<211>    12
<212>    PRT
<213>    Artificial Sequence

<220>
<223>    Pep-4


<400>    2
Glu Val Thr Arg Ala Glu Trp Glu Gln Lys Asp Glu
  1               5                  10


<210>    3
<211>    13
<212>    PRT
<213>    Artificial Sequence

<220>
<223>    Pep-6


<400>    3
Ser Arg Ala Ser Gly Lys Pro Val Asn His Ser Thr Arg
  1               5                  10